(19) Europäisches Patentamt
European Patent Office
Office européen des brevets

(11) EP 0 888 304 B1

(12) **FASCICULE DE BREVET EUROPEEN**

(45) Date de publication et mention de la délivrance du brevet: **04.10.2001 Bulletin 2001/40**

(21) Numéro de dépôt: **97914357.5**

(22) Date de dépôt: **05.03.1997**

(51) Int Cl.$^7$: **C07D 211/88**, C07D 401/04, C07D 409/04, A61K 31/445

(86) Numéro de dépôt international: **PCT/FR97/00388**

(87) Numéro de publication internationale: **WO 97/32852 (12.09.1997 Gazette 1997/39)**

(54) **GLUTARIMIDES 3-ARYL-3-CARBOXYALKYL SUBSTITUES, LEUR PREPARATION PAR CYCLISATION D'ACIDES DICARBOXYLIQUES DE LA 4-ARYL-4-CYANOHEPTANE ET LEUR UTILISATION POUR LA PREPARATION DE 3-ARYL-3-HYDROXYALKYLPIPERIDINES**

3-ARYL-R-CARBOXYALKYL SUBSTITUIERTE GLUTARIMIDE, IHRE HERSTELLUNG DURCH RINGSCHLUSS VON 4-ARYL-4-CYANOHEPTANDICARBONSÄUREN UND IHRE VERWENDUNG ZUR HERSTELLUNG VON 3-ARYL-3-HYDROXYALKYLPIPERIDINEN

SUBSTITUTED 3-ARYL-3-CARBOXYALKYL GLUTARAMIDES, METHOD FOR PREPARING SAME BY 4-ARYLO-4-CYANOHEPTANE DICARBOXYLIC ACID CYCLISATION, AND USE THEREOF FOR PREPARING 3-ARYL-3-HYDROXYALKYLPIPERIDINES

(84) Etats contractants désignés:
**AT BE CH DE DK ES FI FR GB GR IE IT LI LU MC NL PT SE**

(30) Priorité: **07.03.1996 FR 9602880**

(43) Date de publication de la demande: **07.01.1999 Bulletin 1999/01**

(73) Titulaire: **SANOFI-SYNTHELABO**
**75013 Paris (FR)**

(72) Inventeurs:
- **CAMUS, Philippe**
**F-31600 Muret (FR)**
- **DESCAMPS, Marcel**
**F-31600 Le-Lherm (FR)**
- **RADISSON, Joel**
**F-31600 Saubens (FR)**

(74) Mandataire: **Gillard, Marie-Louise et al**
**Cabinet Beau de Loménie**
**158, rue de l'Université**
**75340 Paris Cédex 07 (FR)**

(56) Documents cités:
**EP-A- 0 156 433**
**EP-A- 0 512 901**
**EP-A- 0 591 040**
**EP-A- 0 673 928**
**US-A- 3 985 888**

- **INDIAN J. CHEM. (1973), 11(3), 225-8 CODEN: IJOCAP, 1973, XP000609461 FATEEN, A. K. ET AL: "Hydrolysis of some.gamma.-cyano-.gamma.-arylpimelonitriles"**
- **INDIAN J. CHEM., SECT. B (1980), 19B(2), 122-4 CODEN: IJSBDB;ISSN: 0376-4699, 1980, XP000607818 KADDAH, A. M. ET AL: "Behavior of some.gamma.-cyano-.gamma.-arylpimelonitriles and related compounds towards hydrolysis"**
- **COLLECT. CZECH. CHEM. COMMUN. (1971), 36(9), 3300-13 CODEN: CCCCAK, 1971, XP000608607 JILEK, J. O. ET AL: "Neurotropic and psychotropic agents. XLVIII. Derivatives of phenanthrene, acenaphthene, anthracene, and naphthalene"**
- **CHEMICAL ABSTRACTS, vol. 69, no. 21, 18 Novembre 1968 Columbus, Ohio, US; abstract no. 86577c, SHIRAI, HIDEAKI ET AL: "Structure of the by-products in the Michael reaction of m-methoxybenzyl cyanide and methyl acrylate" XP002018008 & NAGOYA SHIRITSU DAIGAKU YAKUGAKUBU KENKYU NEMPO (1967), 15, 52-6 CODEN: NSDYAI, 1967,**
- **J. ORGANIC CHEMISTRY, vol. 25, 1960, pages 164-174, XP002018007 C. F. KOELSCH: "Syntheses Utilizing .gamma.-Cyano-.gamma.-phenylpimelonitrile"**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

• ANN. CHIM., vol. 51, 1961, ROME, pages 1382-1391, XP000607815

## Description

La présente invention concerne de nouveaux glutarimides 3,3-disubstitués par un groupe aryle et par un groupe carboxy ($C_1$-$C_2$)alkyle, un procédé pour leur préparation par de nouveaux intermédiaires et l'utilisation desdits glutarimides pour la préparation des pipéridines correspondantes, 3,3-disubstituées par le même groupe aryle et par un groupe hydroxy ($C_2$-$C_3$)alkyle.

Le document EP 512 901 décrit des antagonistes des neurokinines qui sont préparés à partir de pipéridines 3,3-disubstituées par un groupe aryle et par un groupe hydroxyalkyle. Ces pipéridines 3,3-disubstituées sont préparées à partir de nitriles par réduction en amines et cyclisation.

Les publications de X. Edmonds-Alt et al., European Joum. Pharmacol., 1993, *250,* 403-413 et Life Sciences 1995, *56* (1), 27-32 décrivent, respectivement, un $NK_1$-antagoniste, le chlorure de (S)1-{2-[3-(3,4-dichlorophényl)-1-(3-isopropoxyphénylacétyl)pipéridin-3-yl]éthyl}-4-phényl-1-azoniabicyclo[2.2.2]octane, ou SR 140333 et un $NK_3$-antagoniste, le (S)-N-[1-[3-{1-benzoyl-3-(3,4-dichlorophényl)pipéridin-3-yl}propyl]-4-phénylpipéridin-4-yl]-N-méthylacétamide ou SR 142801.

La préparation de ces deux produits est décrite, respectivement dans EP 591 040 et EP 673 928. Ces documents décrivent, en tant qu'intermédiaires, des pipéridines 3,3-disubstituées qui peuvent être représentées par la formule (A) suivante :

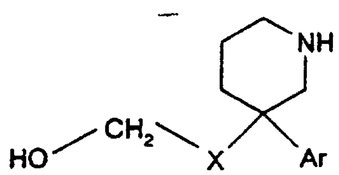

(A)

dans laquelle X est méthylène ou éthylène et Ar représente un phényle non substitué ou substitué une ou plusieurs fois par un des substituants choisis parmi un atome d'halogène, un hydroxyle, un ($C_1$-$C_4$)alcoxy, un trifluorométhyle, un ($C_1$-$C_4$)alkyle, lesdits substituants étant identiques ou différents ; un groupe pyridyle ; un groupe thiényle.

Selon EP 512 901, EP 591 040 et EP 673 928, la préparation des produits finals sous forme optiquement pure comporte la séparation des isomères optiques des composés de formule (A) ci-dessus.

Dans EP 673 928, la préparation d'une pipéridine 3,3-disubstituée de formule (A), dans laquelle X est éthylène et Ar est 3,4-dichlorophényle est réalisée à partir du 3,4-dichlorophénylacétonitrile (i), par action de l'acrylate de méthyle, cyclisation du 4-cyano-4-(3,4-dichlorophényl)heptanedioate de méthyle (ii) en 3-(3,4-dichlorophényl)-3-(2-méthoxycarbonyl)éthyl-2-oxopipéridine (iii), saponification de ce produit pour obtenir l'acide libre correspondant (iv) et réduction de ce dernier, selon le SCHEMA A ci-après.

## SCHEMA A

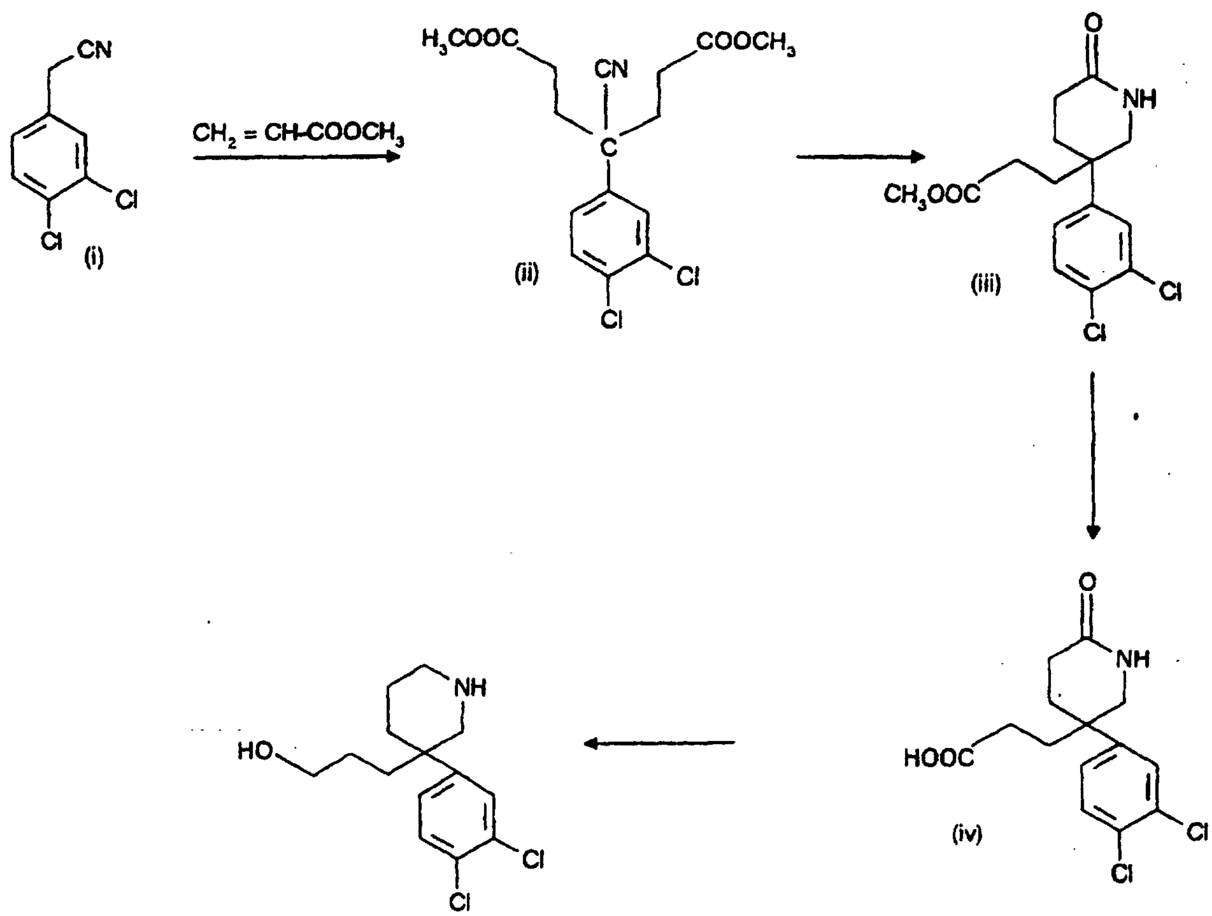


(A : X = $CH_2CH_2$ ; Ar = 3,4-dichlorophényle)

Il a été maintenant trouvé qu'en saponifiant le composé (ii) ci-dessus, on obtient l'acide dicarboxylique correspondant qui cyclise très facilement avec un rendement très élevé pour donner un glutarimide 3,3-disubstitué.

Il a été également trouvé que ce nouveau glutarimide peut être aisément dédoublé et transformé en composé de formule (A) optiquement pur par simple réduction.

Il a également été trouvé que, dans le procédé de préparation de certains desdits glutarimides, il est possible de séparer les isomères optiques en amont, lorsque les intermédiaires présentent un carbone asymétrique.

De façon plus générale, il a été trouvé toute une série de glutarimides 3-carboxylalkyl-3-aryl-disubstitués qui constituent des intermédiaires utiles pour la préparation des pipéridines 3,3-disubstituées de formule (A) ci-dessus. Par rapport aux pipéridines diones décrites dans la demande WO94/21609, ces glutarimides 3,3-disubstituées présentent l'intérêt de pouvoir être dédoublés et utilisés sous forme optiquement active.

La préparation des composés de formule

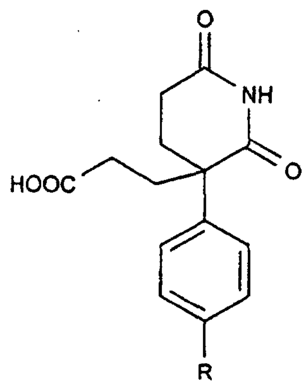

dans laquelle R est un atome de chlore ou un groupe méthoxy est décrite dans Fateen et al., Indian J. Chem., 1973, 11, 225-228.

L'article Kaddah et al., Indian J. Chem., 1980, 19B, 122-124 décrit le même type de composés que la publication précédente, dans lesquels R est un atome de brome ou de fluor ou par un groupe méthyle.

Le brevet US 3,985,888 décrit des dérivés 2,3-dihydro-spiro[naphtalène-1,3'-pipéridine]-2',4,6'-triones à activité sédative de formule :

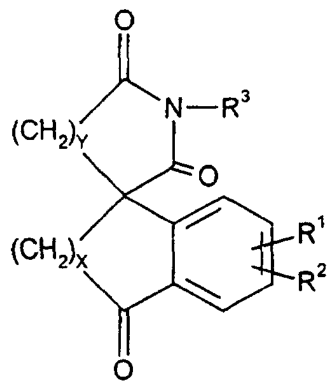

dans laquelle

- $R_1$ et $R_2$ identiques ou différents, représentent hydrogène, halogène, alkyle ou alcoxy inférieurs, nitro ;
- $R_3$ est hydrogène ou alkyle inférieur ;
- X et Y = 1 ou 2.

Les exemples 4, 9, 11 et 17 de préparation de ces composés décrivent respectivement comme intermédiaires l'acide 2,6-dioxo-3-phényl-3-pipéridine propionique ; l'acide 2,6-dioxo-3-(p-tolyl)-3-pipéridine propionique ; le γ-cyano-γ-3,4-diméthoxyphényl-pimélate; l'acide 2,6-dioxo-3-(o-tolyl)-3-pipéridine propionique et l'acide 3-(p-chlorophényl)-2,6-dioxopipéridine propionique.

Un intermédiaire de formule (XXV) est décrit dans Jilek et al. Collect. Czech. Chem. Commun., 1971, 36, 3300-3313:

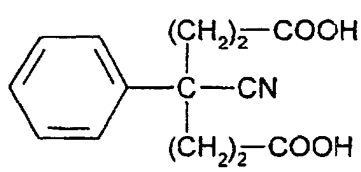 (XXV)

Le Chemical Abstract Shirai et al. 1969, vol. 69, n° 1, n° 486577 décrit un composé

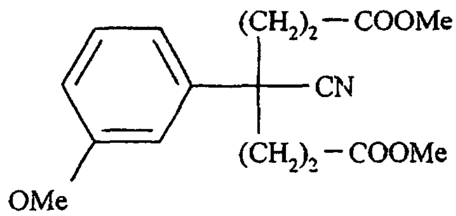

dont l'acide correspondant a été utilisé pour obtenir le dérivé di-tert-butylique.

Un intermédiaire (5) de formule :

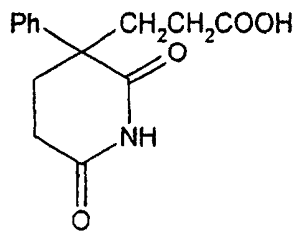

utilisé pour préparer l'acide 3-p-iodophényl-2,6-pipéridinedione-3-propionique est décrit dans Koelsch et al., J. Org. Chem., 1960, 25, 164-174.

L'article Branchini et al., Ann. Chim., 1961, 51, 1382-1391 décrit des dérivés glutarimides de formule générale:

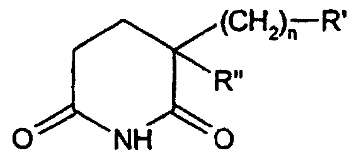

parmi lesquels les dérivés XV : n = 1 ; R' = COOH ; R" = phényle et XVI : n = 2 ; R' = COOH ; R" = phényle.

Les composés mentionnés ci-dessus sont exclus de l'invention.

Ainsi, selon un de ses aspects, l'invention concerne les glutarimides de formule (I):

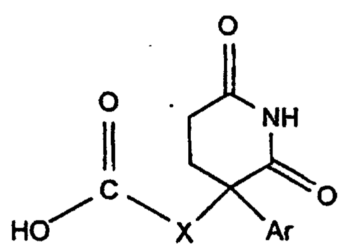

(I)

dans laquelle Ar représente un phényle non substitué ou substitué une ou plusieurs fois par un des substituants choisis parmi un atome d'halogène, un hydroxyle, un $(C_1\text{-}C_4)$alcoxy, un trifluorométhyle, un $(C_1\text{-}C_4)$alkyle, lesdits substituants étant identiques ou différents ; un groupe pyridyle ; un groupe thiényle et X est méthylène ou éthylène et leurs sels, sous réserve que :

- lorsque X est méthylène, Ar est différent d'un phényle non substitué ; et
- lorsque X est éthylène, Ar est différent d'un phényle non substitué ou d'un phényle substitué en position 2 par un méthyle ou en position 4 par un atome de chlore, un atome de brome, un atome de fluor, un méthyle ou un méthoxy.

L'invention concerne également les glutarimides de formule (I) sous forme d'un de ses sels optiquement actifs, d'un de ses énantiomères ou d'un des sels de ceux-ci.

Les glutarimides préférés selon la présente invention sont ceux de formule (I) ci-dessus, dans laquelle X est méthylène ou éthylène et Ar est 3,4-dichlorophényle ou 3,4-difluorophényle, leurs sels et leurs énantiomères.

Les sels des glutarimides de formule (I) peuvent être ceux avec des bases inorganiques ou organiques, par exemple les sels de sodium, de potassium, de calcium, de magnésium, de barium, de zinc, d'argent, de triméthylamine, de triéthylamine, de tris-hydroxyméthylméthanamine (trométhanol), d'éthanolamine, de diéthanolamine, de 1-méthylpipéridine, de 4-méthylmorpholine ou d'une base optiquement active, en particulier une amine optiquement active.

Particulièrement préférés sont les sels des glutarimides de formule (I), dans laquelle X est méthylène ou éthylène et Ar est 3,4-difluorophényle ou 3,4-dichlorophényle, avec des amines optiquement actives.

L'invention concerne également un procédé de préparation des glutarimides de formule (I):

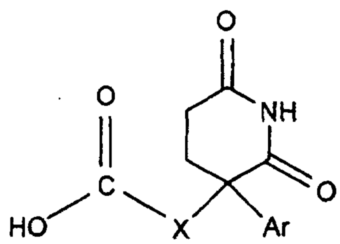

(I)

dans laquelle Ar et X sont tels que définis ci-dessus, ledit procédé étant, selon une première alternative, caractérisé en ce que :

on cyclise un composé de formule (III) :

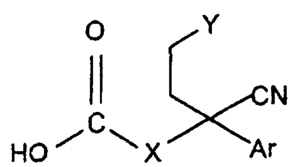

(III)

dans laquelle Ar et X sont tels que définis ci-dessus pour la formule (I) et Y est un groupe cyano ou carboxy ;
et on isole le composé ainsi obtenu de formule (I) sous forme d'un de ses sels ou sous sa forme acide que l'on transforme éventuellement en un de ses sels.

Lorsqu'on souhaite préparer un glutarimide de formule (I) dans laquelle X est méthylène, le composé de départ de formule (III) possède un atome de carbone chiral. Il est donc possible d'utiliser un composé optiquement actif en tant que produit de départ. Dans ce cas, ledit produit de départ peut avoir la formule (III), dans laquelle X est méthylène et Y est cyano et peut être optiquement actif. Un tel composé de départ est particulièrement avantageux pour la préparation des glutarimides de formule (I), dans laquelle X est méthylène, et de leurs sels.

Les composés intermédiaires de formule (III), dans laquelle X est méthylène, Y est cyano et Ar est 3,4-difluorophényle ou 3,4-dichlorophényle sont préférés.

Dans un aspect avantageux du procédé selon l'invention, le composé de formule (III) est obtenu par saponification du ou des groupe(s) ester(s) d'un arvlacétonitrile $\alpha,\alpha$-disubstitué de formule (II) :

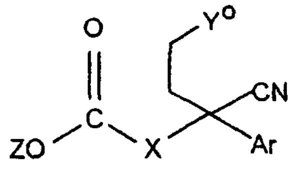

(II)

dans laquelle Ar et X sont tels que définis ci-dessus pour la formule (I), $Y^o$ est un groupe cyano ou COOAlk et Z est l'hydrogène ou Alk, Alk étant un groupe $(C_1\text{-}C_3)$alkyle.

L'invention concerne donc selon une seconde alternative, un procédé de préparation des glutarimides de formule (I) telle que définie ci-dessus, caractérisé en ce que :

(a) on saponifie le ou les groupe(s) ester(s) d'un arylacétonitrile α,α-disubstitué de formule (II) :

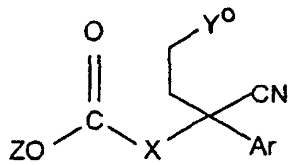

(II)

dans laquelle Ar et X sont tels que définis ci-dessus pour la formule (I), Y° est un groupe cyano ou COOAlk, Z est l'hydrogène ou Alk, Alk étant un groupe $(C_1-C_3)$alkyle, et au moins un des groupes COOZ et Y° étant COOAlk,
(b) on cyclise le composé de formule (III):

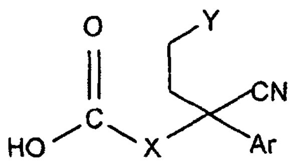

(III)

dans laquelle Ar et X sont tels que définis ci-dessus et Y est un groupe cyano ou carboxy ;

et on isole le composé ainsi obtenu de formule (I) sous forme d'un de ses sels ou sous sa forme acide que l'on transforme éventuellement en un de ses sels.

Lorsqu'on souhaite préparer selon cette deuxième alternative un glutarimide de formule (I) dans laquelle X est éthylène, le composé de départ le plus accessible, et donc particulièrement avantageux, possède la formule (II) dans laquelle X est éthylène, Y° est COOAlk et Z est Alk. Dans ce cas, le composé de départ ne possède pas d'atome de carbone chiral.

Les composés de départ de formule (II), dans laquelle X est éthylène, Y° est COOAlk et Z est Alk sont particulièrement avantageux, ceux de formule (II) dans laquelle X est éthylène, Y° est $COOCH_3$, Z est $CH_3$ et Ar est 3,4-difluorophényle ou 3,4-dichlorophényle sont préférés.

Le procédé selon la présente invention (1ère et 2ème alternatives) est illustré dans le SCHEMA 1 ci-après.

## SCHEMA 1

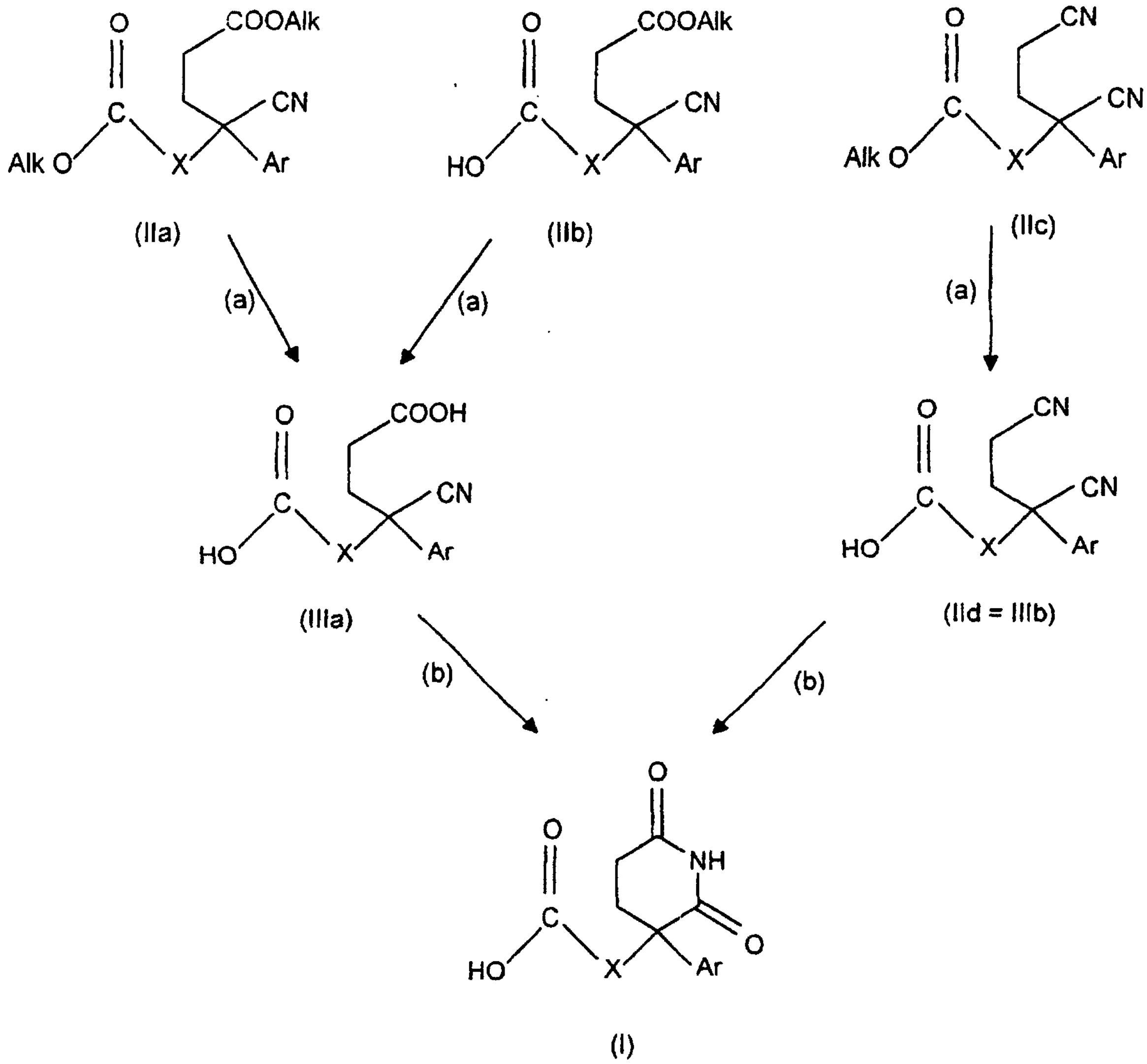


(IIa) (IIb) (IIc)

(IIIa) (IId = IIIb)

(I)

L'étape (a) du procédé de la présente invention (2ème alternative) consiste en une saponification du groupe ester présent dans le composé de formule (II), notamment, selon le SCHEMA 1, la saponification d'un composé choisi parmi ceux représentés par les formules (IIa), (IIb) et (IIc).

La saponification est conduite de préférence en utilisant un hydroxyde ou un carbonate alcalin. La réaction a lieu en milieu hydroalcoolique ou dans un mélange tétrahydrofurane/eau.

Le composé de formule (III) est isolé par acidification avec un acide minéral ou organique comme l'acide sulfurique, l'acide bromhydrique, l'acide méthanesulfonique ou, de préférence, l'acide chlorhydrique jusqu'à une valeur de pH acide, qui peut varier entre 0 et 3, pH auquel le produit précipite.

Dans l'étape (b) qui caractérise la 1ère alternative du procédé de la présente invention, la cyclisation du composé (III) est effectuée par hydratation en présence d'un acide choisi parmi l'acide phosphorique, l'acide chlorhydrique ou préférentiellement avec de l'acide sulfurique concentré. On peut également utiliser un solvant non protique tel que par exemple le toluène dans ce cas en présence d'acide sulfonique tel que par exemple l'acide paratoluènesulfonique monohydraté ou méthanesulfonique en présence d'eau. La réaction est conduite dans un solvant protique tel que par exemple dans l'acide acétique chauffé à reflux. Après 1 à 2 heures de chauffage, la réaction est complète et le glutarimide de formule (I) ainsi obtenu est isolé selon les méthodes conventionnelles. En général, il suffit de laisser refroidir le mélange réactionnel pour séparer le glutarimide après précipitation ou de verser ledit mélange réactionnel dans l'eau pour faire précipiter le produit final.

Le glutarimide de formule (I) ainsi obtenu peut être isolé soit sous forme d'acide libre directement du mélange

réactionnel, ou bien il peut être isolé sous forme d'un de ses sels par traitement du mélange réactionnel avec la base choisie.

On peut également transformer l'acide libre en un de ses sels, notamment avec une base organique optiquement active, de façon à isoler un sel diastéréoisomérique du composé de formule (I) qui, par neutralisation, donne un des deux énantiomères.

Le procédé de la présente invention, selon l'une ou l'autre de ses alternatives, permet également de séparer les isomères optiques de certains intermédiaires (II) et (III), notamment des composés ayant les formules (IIb), (IIIa) et (IIIb), de façon à effectuer cette séparation bien en amont du produit final que l'on entend obtenir. Les réactions qui conduisent au composé (I) ne provoquent par ailleurs pas de racémisation.

Cette possibilité confère au procédé de la présente invention un grand avantage par rapport aux procédés connus pour la préparation de la pipéridine 3,3-disubstituée de formule (A).

Les composés de départ de formule (II) sont soit connus, soit peuvent être préparés selon des méthodes connues à partir d'un arylacétonitrile de formule $ArCH_2CN$.

Pour préparer les composés de formule (II) dans laquelle X est méthylène, la méthode générale prévoit la réaction d'un arylacétonitrile de formule $ArCH_2CN$, dans laquelle Ar est tel que défini ci-dessus, avec un acide haloacétique ou un de ses esters de formule (IV):

$$Hal\text{-}CH_2\text{-}COOZ \qquad (IV)$$

dans laquelle Z est tel que défini ci-dessus et Hal représente un halogène, de préférence le chlore ou le brome, ou encore par estérification d'un cyanoacide de formule (V) :

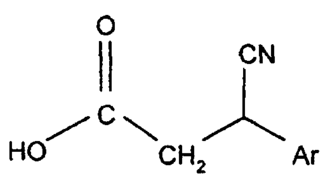

((V) avec Z = H)

dans laquelle Z représente l'hydrogène, dans les conditions décrites, pour Ar égal à 3,4-dichlorophényle, dans EP 612 716. Le produit ainsi obtenu, de formule (V) :

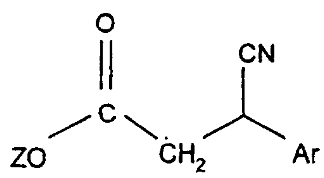

(V)

dans laquelle Z et Ar sont tels que définis ci-dessus, est traité avec un dérivé acrylique de formule (VI) :

$$CH_2 = CH\text{-}Y^{o} \qquad (VI)$$

dans laquelle Y° est tel que défini ci-dessus, dans les conditions bien connues de la condensation acrylique, pour obtenir ainsi le produit souhaité.

Une telle préparation avec des variantes éventuelles est illustré dans le SCHEMA 2 ci-après.

## SCHEMA 2

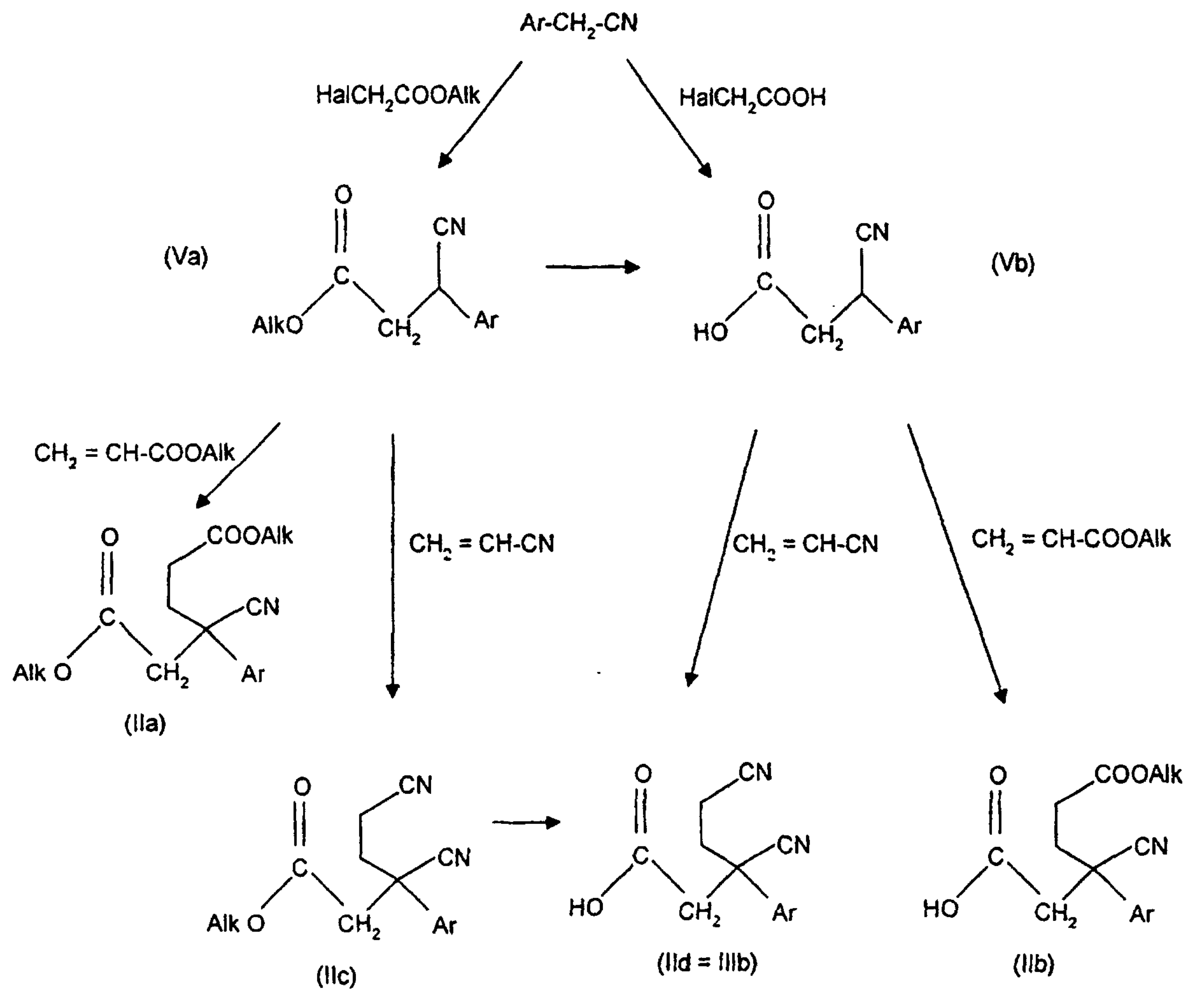


(Va) (Vb) (IIa) (IIc) (IId = IIIb) (IIb)

Pour préparer les composés de formule (II) dans laquelle X est éthylène, on peut suivre la méthode illustrée dans le SCHEMA 2 en utilisant un β-halopropionate d'alkyle au lieu de l'haloacétate correspondant.

Il est cependant préférable d'appliquer la méthode de la condensation acrylique directement sur l'arylacétonitrile de formule $ArCH_2CN$, en utilisant un acrylate en ($C_1$-$C_3$)alkyle dans les conditions décrites, pour Ar égal à 3,4-dichlorophényle, dans EP 673 928, selon le SCHEMA 3 ci-après.

Le composé (IIb) ci-dessus peut également être obtenu sous forme de diacide par saponification de l'ester correspondant.

Toutefois la préparation de l'ester (IIb) ne conduit pas à un intermédiaire primordial car la cyclisation en glutarimide s'effectue soit à partir d'une fonction nitrile par réaction sur un acide soit entre deux fonctions nitrile.

Le composé (IIb) devrait donc dans tous les cas être saponifié.

Le composé (IId), dans les conditions opératoires utilisées selon la présente invention conduit préférentiellement à la cyclisation entre les deux fonctions nitriles donc à la formation des glutarimides préférentiellement à celle de la succinimide qui serait le résultat de la cyclisation entre les fonctions acide et nitrile.

## SCHEMA 3

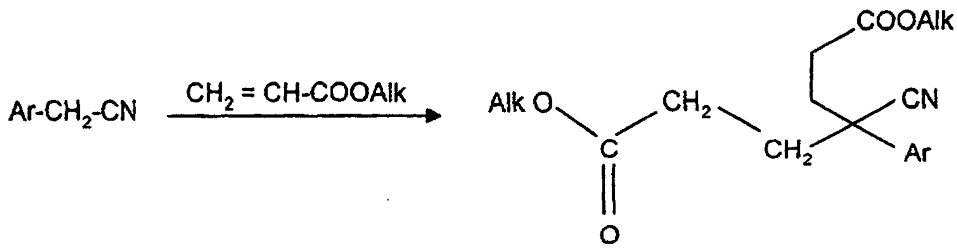

(IIa, X = $CH_2CH_2$)

Par conséquent, selon la 2è alternative du procédé de la présente invention, les produits de départ préférentiels sont ceux de formule (IId) lorsque X est méthylène et ceux de formule (IIa) lorsque X est éthylène.
Les composés de formule (III), qui cyclisent avec des rendements excellents pour donner les glutarimides de la présente invention, sont des intermédiaires clé dans le procédé.
Parmi les composés de formule (III), les composés de formule (III'):

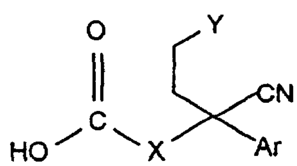

(III')

dans laquelle Ar représente un phényle non substitué ou substitué une ou plusieurs fois par un des substituants choisis parmi un atome d'halogène, un hydroxyle, un $(C_1-C_4)$alcoxy, un trifluorométhyle, un $(C_1-C_4)$alkyle, lesdits substituants étant identiques ou différents ; un groupe pyridyle ; un groupe thiényle, X est méthylène ou éthylène, et Y est un groupe cyano ou carboxy, et leurs sels, sous réserve que :

- lorsque X est éthylène, Ar est différent d'un phényle non susbtitué ; et
- lorsque X est éthylène, Ar est différent d'un phényle non substitué ou d'un phényle substitué en position 2 par un méthyle ou en position 4 par un atome de chlore, un atome de brome, un atome de fluor, un méthyle ou un méthoxy ; ou d'un phényle substitué en position 3 par un méthoxy, ou 3,4-disubstitué par un méthoxy.

L'invention concerne également les composés de formule (III)' sous forme d'un de leurs sels optiquement actifs, d'un de leurs énantiomères ou d'un sel de ceux-ci.
Parmi ces composés de formule (III'), ceux dans lequel Ar est 3,4-difluorophényle ou 3,4-dichlorophényle, ainsi que leurs sels, sont particulièrement avantageux. Les sels de ces produits avec des amines optiquement actives sont préférés. L'acide 3-(3,4-dichlorophényl)-3,5-dicyanopentanoïque et ses sels avec des amines optiquement actives, notamment avec la l-cinchonidine, sont également très avantageux. L'acide (-)-3-(3,4-dichlorophényl)-3,5-dicyanopentanoïque est l'intermédiaire clé pour la préparation du (-)-3-carboxyméthyl-3-(3,4-dichlorophényl)-2,6-dioxo-pipéridine et constitue donc un intermédiaire particulièrement avantageux.
Un autre intermédiaire particulièrement avantageux, utile pour la préparation du 3-carboxyéthyl-3-(3,4-dichlorophényl)-2,6-dioxopipéridine, est l'acide 4-cyano-4-(3,4-dichlorophényl)-3,5-dicyanopentanoïque, tel quel ou sous forme d'un de ses sels.
Les glutarimides de formule (I) ainsi que leurs sels, notamment ceux avec des amines optiquement actives, sont utiles pour la préparation des pipéridines 3,3-disubstituées de formule (A).
Lorsque lesdits glutarimides sont sous forme optiquement active, ils sont utiles pour la préparation des pipéridines 3,3-disubstituées de formule (A) correspondantes, optiquement actives, car la transformation n'est pas racémisante.
La présente invention a donc pour objet, selon un autre de ses aspects, l'utilisation des glutarimides de formule (I) :

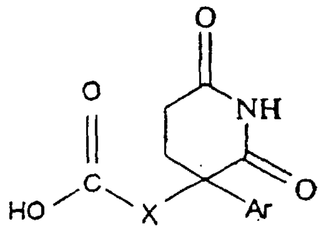

(I)

telle que définie plus haut, de ses sels et énantiomères, pour la préparation de pipéridines 3,3-disubstituées de formule (A) et de leurs sels.

Ladite préparation a lieu par réduction des glutarimides, pendant laquelle les deux groupes carbonyle du glutarimide et le groupe carbonyle de l'acide carboxylique se transforment en même temps en les groupes méthylène correspondants.

Ainsi, selon un autre de ses aspects, la présente invention a pour objet un procédé pour la préparation de pipéridines 3,3-disubstituées de formule (A) et de leurs sels avec des acides inorganiques ou organiques, caractérisé en ce qu'on soumet à réduction un glutarimide correspondant de formule (I) telle que définie ci-dessus, et on isole ladite pipéridine sous forme de base ou d'un de ses sels ou on transforme la base libre en un de ses sels.

Les agents de réductions utilisés sont des complexes du borane tels que par exemple le borane-tétrahydrofurane ou le borane-diméthylsulfure ou encore un hydrure mixte alcalin tel que l'hydrure de lithium aluminium ou l'hydrure de sodium bis (2-méthoxyéthoxy)aluminium en solution dans le toluène (Red-Al® ). Ces réductions se font sans-racémisation, l'agent de réduction préféré est un complexe du borane.

La réduction avec le borane est conduite dans un solvant de préférence aprotique tel que le tétrahydrofurane à la température de reflux du solvant. En général, après 1 à 6 heures de chauffage, la réduction est complète et la pipéridine 3,3-disubstituée est isolée, selon les méthodes conventionnelles, en détruisant d'abord l'excès de borane avec du méthanol. La base libre peut être isolée par évaporation du solvant, reprise du résidu par de l'eau, acidification avec de l'acide chlorhydrique, traitement avec une base, de préférence l'hydroxyde de sodium, et extraction avec un solvant.

La base libre de formule (A) peut être transformée en un de ses sels selon les techniques bien connues. Le borane utilisé pour la réduction peut être généré *in situ* selon des méthodes classiques.

Le glutarimide de formule (I) utilisé pour la préparation de la pipéridine 3,3-disubstituée correspondante de formule (A) peut être sous forme racémique ou sous forme optiquement active.

Lorsque le glutarimide a la formule (I) dans laquelle X est éthylène et qu'il est préparé, selon la voie préférentielle, par cyclisation du diacide (IIIa), celui-ci ne présente pas de carbone chiral et, par conséquent, ledit glutarimide est forcément racémique. Dans ce cas, la séparation des isomères optiques peut être effectuée soit sur le glutarimide, soit sur la pipéridine 3,3-disubstituée. De toute façon, la réduction avec le borane est pratiquement quantitative et l'opérateur peut choisir une des deux alternatives avec le même résultat en termes de rendements.

En outre, la saponification du diester (IIa) (X = éthylène) et la cyclisation du diacide (IIIa) (X = éthylène) se déroulent avec des rendements excellents, ce qui rend le procédé de la présente invention particulièrement avantageux par rapport à celui décrit dans EP 673 928.

Les'exemples suivants illustrent l'invention.

Les points de fusion ont été mesurés avec un appareil de Tottoli. Les déplacements chimiques des spectres RMN sont exprimés en ppm.

**PREPARATION I - *Composé (IIa)***

4-Cyano-4-(3,4-dichlorophényl)heptanedioate de méthyle, *Composé (IIa. 1)*

A une solution de 187 g de 3,4-dichlorophénylacétonitrile dans 250 ml de tétrahydrofurane à reflux contenant 4 ml de Triton B on ajoute progressivement 175 g d'acrylate de méthyle et chauffe encore 30 minutes à reflux. Après réaction, le tétrahydrofurane est distillé, le concentrat est redissous dans 1 litre de toluène et la solution est lavée par 400 ml d'acide chlorhydrique dilué puis par 2 x 150 ml d'eau. Le toluène est distillé et le résidu est cristallisé dans 500 ml de cyclohexane. Le produit attendu est filtré, rincé au cyclohexane et séché à 45°C à l'étuve ventilée pour foumir 340 g du diester attendu ; rendement 95% ; F = 84-85°C. (formule (IIa), X = $CH_2CH_2$, Alk = $CH_3$, Ar = 3,4-dichlorophényle).

$^1$H RMN 200 MHz, DMSO : 2,30 (s, 6H) ; 3,50 (s, 6H) ; 2,08 (m, 2H) ; 7,4 (Ar, 1H) ; 7,7 (Ar, 2H).

En remplaçant l'acrylate de méthyle par les quantités équimoléculaires d'acrylate d'éthyle et d'acrylate de

propyle, on peut obtenir :

- le 4-cyano-4-(3,4-dichlorophényl)heptanedioate d'éthyle, *Composé (IIa.2)* et, respectivement,
- le 4-cyano-4-(3,4-dichlorophényl)heptanedioate de propyle, *Composé (IIa.3).*

**PREPARATION II - *Composé (IIa)***

4-Cyano-4-(3,4-difluorophényl)heptanedioate de méthyle, *Composé (IIa.4)*

En opérant comme décrit dans la PREPARATION I, et en remplaçant le 3,4-dichlorophénylacétonitrile par le 3,4-dlfluorophénylacétonitrile on obtient le 4-cyano-4-(3,4-difluorophényl)heptanedioate de méthyle (formule (IIa), X = $CH_2CH_2$, Alk = $CH_3$, Ar = 3,4-difluorophényle)

De la même façon, en remplaçant l'acrylate de méthyle par les acrylates d'éthyle et d'isopropyle, on peut obtenir, respectivement :

- le 4-cyano-4-(3,4-difluorophényl)heptanedioate d'éthyle, *Composé (IIa.5)*
- le 4-cyano-4-(3,4-difluorophényl)heptanedioate d'isopropyle, *Composé (IIa.6)*

**PREPARATION III - *Composé (IIa)***

En opérant comme décrit dans la PREPARATION I, en partant du 3-pyridineacétonitrile, du 2-thiénylacétonitrile ou du 3-thiénylacétonitrile, on peut obtenir:

le 4-cyano-4-(3-pyridyl)heptanedioate de méthyle, *Composé (IIa.7)*
le 4-cyano-4-(2-thiényl)heptanedioate de méthyle, *Composé (IIa.8)*
le 4-cyano-4-(3-thiényl)heptanedioate de méthyle, *Composé (IIa.9).*

**PREPARATION IV - *Composé (IIa)***

Acide 3-cyano-3-(3,4-dichlorophényl)propionique, *Composé (IIa. 10)*

Dans un ballon, on introduit sous azote à 20°C, 109 g de 3,4-dichlorophénylacétonitrile solubilisé dans 300 ml de tétrahydrofurane. On coule ensuite à -10°C, 240 ml de lithium diisopropylamide 2M. L'addition terminée, on introduit 68 g de chloroacétate de sodium, à la température de 15°C. Le mélange réactionnel est agité pendant trois heures puis on additionne 350 ml de *tert*-butylméthyléther. On coule ensuite le mélange réactionnel dans 300 ml d'eau et 150 g de sulfate d'ammonium ainsi que 400 ml d'eau et 175 g de bisulfate d'ammonium. On soutire la phase aqueuse et la phase organique est successivement lavée à l'eau, séchée et concentrée sous vide pour fournir le cyanoacide ; Rendement 93 %.

**PREPARATION V - *Composé (IIa)***

3-Cyano-3-(3,4-dichlorophényl)propionate de méthyle

(a) A une solution de 260 g de l'acide 3-cyano-3-(3,4-dichlorophényl) propionique dans 2,7 litres d'acétonitrile, on ajoute 320 g de carbonate de potassium. La suspension est chauffée à reflux en additionnant progressivement 110 ml de diméthylsulfate. On laisse revenir le mélange à température ambiante après 15 minutes. Le mélange réactionnel est concentré puis repris par 2 litres de dichlorométhane. La phase organique est lavée à l'eau puis concentrée. Le résidu est repris par 500 ml de méthanol pour fournir l'ester attendu avec un rendement de 80 %.

3-Cyano-3-(3,4-dichlorophényl)hexanedioate de méthyle, *Composé (IIa.11)*

(b) A une solution de 20 g du cyanoester préparé précédemment selon la PREPARATION IV dans 100 ml de tétrahydrofurane on ajoute 7 ml de triton B. On additionne en 30 minutes, 20 ml d'acrylate d'éthyle sur le mélange réactionnel à 66°C. Le mélange réactionnel est concentré, repris par 60 ml de dichlorométhane et lavé par 3 fois 60 ml d'eau. Le cyanodiester est isolé par cristallisation dans 80 ml de méthanol. (Formule (IIa), X = $CH_2$, Alk = $CH_3$, Ar = 3,4-dichlorophényle).

**PREPARATION VI - *Composé (IIa)***

(a) 3-Cyano-3-(3,4-difluorophényl)propionate de méthyle

En opérant comme décrit dans la PREPARATION IV à partir du 3,4-difluorophénylacétonitrile, on obtient le 3-cyano-3-(3,4-difluorophényl) propionate de méthyle.

(b) 3-Cyano-3-(3,4-difluorophényl)hexanedioate de méthyle, *Composé (IIa.11)*

A partir du 3-cyano-3-(3,4-difluorophényl)propionate de méthyle obtenu dans l'étape (a), par réaction avec l'acrylate de méthyle dans les mêmes conditions que celles de la PREPARATION V(b), on peut obtenir le 3-cyano-3-(3,4-difluorophényl)hexanedioate de méthyle.

**EXEMPLE 1**

(a) Acide 4-cyano-4-(3,4-dichlorophényl)heptanedioïque

- A partir d'un diester

  Dans un ballon, on introduit 358,2 g du diester obtenu dans la PREPARATION 1 et 1,5 litre de méthanol puis on ajoute 250 ml d'hydroxyde de sodium à 30 % et 100 ml d'eau. Le mélange devient limpide et est abandonné sous agitation pendant 1 heure et 30 minutes à température ambiante jusqu'à disparition de l'ester. On élimine alors le méthanol sous vide et on obtient un résidu huileux qui est repris dans 1,5 litre d'eau, puis successivement refroidi dans un bain de glace et acidifié avec de l'acide sulfurique jusqu'à ce que des cristaux blancs précipitent à pH < 3. Le diacide est séparé par filtration, essoré et rincé à l'eau (pH = 5) puis séché à l'étuve ventilée à poids constant pour fournir 326 g du produit attendu.
- A partir d'un nitrile

  A une solution de 187 g de 3,4-dichlorophénylacétonitrile dans 250 ml de tétrahydrofurane à ébullition contenant 4 ml de Triton B on ajoute progressivement 175 g d'acrylate de méthyle et chauffe encore 30 minutes à reflux. Après réaction, le tétrahydrofurane est distillé, le résidu est solubilisé dans 1,5 litre de méthanol et additionné d'une solution d'hydroxyde de sodium 30%. Le mélange réactionnel est agité pendant 1 heure à température ambiante jusqu'à disparition de l'ester, puis le méthanol est distillé. Le résidu est solubilisé dans 1,5 litre d'eau, acidifié à pH < 3 par de l'acide sulfurique dilué et laissé cristalliser 30 minutes. Le diacide est filtré, rincé à l'eau à neutralité (pH 5) et séché à l'étuve ventilée à 60°C à poids constant pour fournir 314 g de produit attendu ; Rendement : 95%. (Formule (IIIa), X = $CH_2CH_2$, Ar = 3,4-dichlorophényle)

  $^1$H RMN 200 MHz, DMSO : 2,0 (m, 2H) ; 2,29 (s, 6H) ; 7,4 (Ar, 1H) ; 7,65 (Ar, 2H) ; 11,5 (COO<u>H</u>, 2H).

(b) 3-(2-Carboxyéthyl)-3-(3,4-dichlorophényl)-2,6-dioxopipéridine racémique

330 g d'Acide 4-cyano-4-(3,4-dichlorophényl)heptanedicarboxylique obtenus précédemment sont chauffés à reflux pendant 30 minutes dans 1 litre d'acide acétique contenant 50 g d'acide sulfurique concentré. Après réaction, le mélange réactionnel est laissé cristalliser et le produit attendu est filtré, rincé à l'acide acétique puis au t-butylméthylether jusqu'à ce que le papier Congo ne bleuisse plus (pH > 3). Le produit est séché à l'étuve ventilée à 70°C à poids constant pour fournir 306,5 g du produit attendu ; R = 92% ; F = 234°C.

$^1$H RMN 200 MHz, DMSO : 2,0 et 2,5 (m, 8H) ; 7,25 (Ar, 1H) ; 7,5-7,6 (Ar, 2H) ; 11,0 (s, N<u>H</u>) ; 12 (COO<u>H</u>, 1H).

**EXEMPLE 2**

Sel de quinine de (+)-3-(2-carboxyéthyl)-3-(3,4-dichlorophényl)-2,6-dioxo-pipéridine

100 g du composé racémique préparé précédemment solubilisés dans 1 litre d'ammoniaque 0,5 M sont additionnés de 65,4 g de quinine en solution dans 1 litre de méthanol. Le produit qui a cristallisé est filtré et lavé par un mélange eau/méthanol 75/25 puis recristallisé d'un mélange méthanol/eau 50/50. On obtient après séchage 78,7 g de sel de quinine de la (+) 3-(2-carboxyéthyl)-3-(3,4-dichlorophényl)-2,6-dioxopipéridine, pureté énantiomérique 97 % déterminée par HPLC après dérivatisation par la (S) méthylbenzylamine.

**EXEMPLE 3**

(+)-3-(2-Carboxyéthyl)-3-(3,4-dichlorophényl)-2,6-dioxopipéridine

70 g de sel de quinine obtenus selon l'EXEMPLE 2 sont solubilisés dans un mélange de 1,3 litre d'acétate d'éthyle et 1,3 litre d'acide chlorhydrique 1N. La phase aqueuse est écartée, la phase organique est séchée sur sulfate de sodium et concentrée à sec. Le résidu est cristallisé dans 200 ml d'éther de pétrole et séché à 40°C. F = 201°C.

$$[\alpha]_D^{20} = +137{,}1 \ (c = 1, CH_3OH)$$

**EXEMPLE 4**

(a) Acide 4-cyano-4-(3,4-difluorophényl)heptanedioïque

Par saponification du diester méthylique obtenu dans la PREPARATION II et selon les conditions décrites dans l'EXEMPLE 1(a), on peut obtenir l'acide 4-cyano-4-(3,4-difluorophényl)heptanedioïque (Formule (IIIa), X = $CH_2CH_2$, Ar = 3,4-difluorophényle).

(b) 3-(2-Carboxyéthyl)-3-(3,4-difluorophényl)-2,6-dioxopipéridine

La cyclisation du diacide obtenu dans l'étape (a), par déshydratation avec l'acide sulfurique à 80 % dans l'acide acétique dans les conditions de l'EXEMPLE 1(b), fournit le produit attendu (Formule I, X = $CH_2CH_2$, Ar = 3,4-difluorophényle).

**EXEMPLE 5**

(a) Acide 3,5-dicyano-3-(3,4-dichlorophényl)pentanoïque

Dans un ballon, on introduit une solution dans le dichlorométhane de 122 g du cyanoacide préparé précédemment puis on additionne goutte à goutte 500 ml d'une solution d'hydroxyde de sodium 2N en maintenant la température à 15°C. L'addition terminée, on laisse décanter puis on sépare la phase organique. La phase organique est versée dans un ballon, puis sous agitation on additionne en 30 minutes 50 ml d'acrylonitrile tout en maintenant la température inférieure à 20°C, puis on ajoute à nouveau 5 ml d'acrylonitrile. Le mélange réactionnel est agité pendant trente minutes puis refroidi à 10°C et additionné goutte à goutte de 500 ml d'acide chlorhydrique 2N. On laisse le mélange sous agitation, puis on sépare un précipité par filtration, qui après rinçage à l'eau et séchage fournit 121 g du dinitrile attendu (Formule (IIIb) ou (IId), X = $CH_2$, Ar = 3,4-dichlorophényle); F = 170°C.

(b) 3-Carboxyméthyl-3-(3,4-dichlorophényl)-2,6-dioxopipéridine

Dans un ballon, on introduit 20 g du dérivé préparé selon (a) dans 80 ml d'acide acétique. On additionne alors goutte à goutte 6,75 ml d'acide sulfurique à 80% puis on chauffe le mélange réactionnel à 80°C puis à reflux pendant 1 heure et 30 minutes. On laisse revenir le mélange réactionnel à température ambiante puis on additionne 240 ml d'eau glacée dans celui-ci. Après agitation à 10°C pendant 30 minutes, on sépare le précipité par filtration, on le reprend dans 130 ml d'acide acétique que l'on distille ensuite pour obtenir un résidu qui cristallise pour fournir 17 g du produit attendu (Formule (I), X = $CH_2$, Ar = 3,4-dichlorophényle) ; F = 203°C.

**EXEMPLE 6**

Sel de cinchonidine de l'acide (-)-3,5-dicyano-3-(3,4-dichlorophényl) pentanoïque

Dans un tricol avec agitation mécanique, on introduit 50 g du produit de l'EXEMPLE 3(a) dans 375 ml de méthanol. On ajoute ensuite 24,8 g de l-cinchonidine et chauffe le mélange réactionnel à reflux pendant une heure puis on le laisse refroidir et on sépare un précipité par filtration qui après séchage fournit 31,1 g du produit attendu (Formule (III), X = $CH_2$, Y = COOH, Ar = 3,4-dichlorophényle, isomère (-)) ; F = 199,5°C.

**EXEMPLE 7**

Acide (-)-3,5-dicyano-3-(3,4-dichlorophényl)pentanoïque

Dans un tricol avec agitation mécanique on met en suspension 15 g du sel de cinchonidine préparé précédemment dans 75 ml d'eau puis on additionne goutte à goutte 9,5 ml d'acide chlorhydrique 6N en maintenant la température du mélange réactionnel à 20°C dans un bain de glace tout en agitant pendant 30 minutes. On sépare un précipité par filtration qui est ensuite rincé à l'eau et séché sous vide pour fournir 7,96 g du composé attendu (Formule (IIIb), X = $CH_2$, Ar = 3,4-dichlorophényl, isomère (-)) ;

$$[\alpha]_D^{20} = -2 \text{ (c = 1, } CH_3OH)$$

Enantiomère (+) :

$$[\alpha]_D^{20} = +2 \text{ (c = 1, } CH_3OH)$$

**EXEMPLE 8**

(+)-3-Carboxyméthyl-3-(3,4-dichlorophényl)-2,6-dioxopipéridine

En procédant selon l'EXEMPLE 5(b) à partir de l'acide obtenu à l'EXEMPLE 7, on obtient le composé optiquement pur ci-dessus, F = 229°C.

$$[\alpha]_D^{20} = +131{,}5 \text{ (c = 1, } CH_3OH)$$

Enantiomère (-) :

$$[\alpha]_D^{20} = -131{,}5 \text{ (c = 1, } CH_3OH)$$

**EXEMPLE 9**

(a) Acide 3-cyano-3-(3,4-dichlorophényl)hexanedioïque

A une suspension de 11 g du cyanodiacide préparé précédemment selon l'EXEMPLE 7, dans 34 ml de méthanol, on additionne 33 ml d'hydroxyde de sodium 4N. Après 30 minutes à 40°C, le mélange réactionnel est concentré, repris par 42 ml d'eau et acidifié par de l'acide chlorhydrique jusqu'à un pH < 3. L'acide attendu précipite de la phase aqueuse. Après filtration et séchage, on isole 9,9 g du composé attendu ; F = 152°C ; Rendement 98 %.

(Formule (IIIa), X = $CH_2$, Ar = 3,4-dichlorophényle).

(b) 3-Carboxyméthyl-3-(3,4-dichlorophényl)-2,6-dioxopipéridine

A partir du diacide obtenu dans l'étape (a), par cyclisation dans les conditions décrites dans l'EXEMPLE 1 (b), on obtient le produit attendu, identique à celui de l'EXEMPLE 5(b).

**EXEMPLE 10**

Utilisation de la 3-carboxyméthyl-3-(3,4-dichlorophényl)-2,6-dioxo-pipéridine pour la préparation de la 3-(3,4-dichlorophényl)-3-(2-hydroxyéthyl)pipéridine.

Dans un ballon muni d'une agitation mécanique, on introduit 58,0 g du composé préparé précédemment dans 600 ml de tétrahydrofurane puis on additionne lentement et goutte à goutte 800 ml d'une solution 1,0 M de borane dans le tétrahydrofurane. Le mélange réactionnel est ensuite chauffé par paliers jusqu'au reflux pendant 5 heures. On laisse revenir ensuite la température à température ambiante puis on additionne lentement 300 ml de méthanol. Le

mélange réactionnel est ensuite concentré sous vide pour fournir un résidu qui est repris dans l'eau puis successivement on acidifie la phase aqueuse avec une solution d'acide chlorhydrique à 35 %, on chauffe à reflux sous agitation pendant 1 heure puis on ramène à température ambiante. On alcalinise jusqu'à pH = 14 par addition d'une solution d'hydroxyde de sodium concentrée puis on extrait avec 400 ml de dichlorométhane. Les phases organiques sont rassemblées puis successivement lavées à l'eau, séchées sur $Na_2SO_4$, filtrées et concentrées sous vide pour fournir un résidu qui après séchage conduit à 46,4 g du produit attendu (Formule A, X = $CH_2$, Ar = 3,4-dichlorophényle) ; F = 122°C.

**EXEMPLE 11**

Utilisation de la (+)-3-(2-carboxyéthyl)-3-(3,4-dichlorophényl)-2,6-dioxopipéridine pour la préparation de la (+)-3-(3,4-dichlorophényl)-3-(3-hydroxypropyl)pipéridine

33,0 g du produit préparé précédemment selon l'EXEMPLE 3 dans 120 ml de tétrahydrofurane sont additionnés entre 0 et 20°C de 110 ml d'une solution de borane dans le tétrahydrofurane 1 M. Lorsque l'exothermicité s'arrête, le mélange réactionnel est chauffé à 40°C, puis est additionné de 340 ml de borane dans le tétrahydrofurane 1 M et est chauffé pendant 2 heures à reflux. L'excès de borane est détruit par l'addition progressive de 100 ml de méthanol. Le mélange réactionnel est concentré à sec, additionné de 400 ml d'acide chlorhydrique 6N et chauffé pendant 30 minutes à reflux. Après retour à température ambiante, le mélange est alcalinisé à pH 14 par une solution d'hydroxyde de sodium et extrait par 200 ml de butanol. La phase organique est lavée à l'eau, concentrée au maximum sous vide, reprise par du toluène et à nouveau concentrée à sec sous vide. Le concentrat est repris dans du dichlorométhane, additionné de chlorure d'hydrogène anhydre, évaporé à sec et concrétisé dans l'acétonitrile pour fournir 26,2 g du produit attendu (Formule A, X = $CH_2CH_2$, Ar = 3,4-dichlorophényle).

$$[\alpha]_D^{20} = +6,5 \ (c = 1, CH_3OH)$$

On prépare également le camphosulfonate de ce dérivé par réaction avec l'acide camphosulfonique ;

$$[\alpha]_D^{20} = +23,0 \ (c = 1, CH_3OH)$$

## Revendications

1. Glutarimide de formule (I):

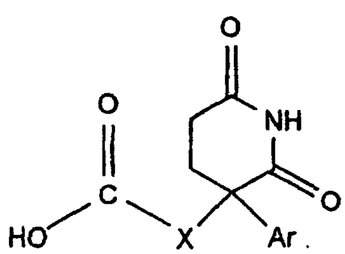

(I)

dans laquelle Ar représente un phényle non substitué ou substitué une ou plusieurs fois par un des substituants choisis parmi un atome d'halogène, un hydroxyle, un ($C_1$-$C_4$)alcoxy, un trifluorométhyle, un ($C_1$-$C_4$)alkyle, lesdits substituants étant identiques ou différents ; un groupe pyridyle ; un groupe thiényle et X est méthylène ou éthylène, et ses sels, sous réserve que :

- lorsque X est méthylène, Ar est différent d'un phényle non substitué ; et
- lorsque X est éthylène, Ar est différent d'un phényle non substitué ou d'un phényle substitué en position 2 par un méthyle ou en position 4 par un atome de chlore, un atome de brome, un atome de fluor, un méthyle ou un méthoxy.

2. Glutarimide de formule (I) selon la revendication 1 sous forme d'un de ses sels optiquement actifs, d'un de ses énantiomères, ou d'un sel de ceux-ci.

**3.** Glutarimide selon les revendications 1 ou 2, **caractérisé en ce que** dans la formule (I) Ar est 3,4-difluorophényle ou 3,4-dichlorophényle.

**4.** Glutarimide selon l'une des revendications 1 à 3 sous forme d'un de ses sels avec des amines optiquement actives.

**5.** Procédé de préparation d'un glutarimide de formule (I) :

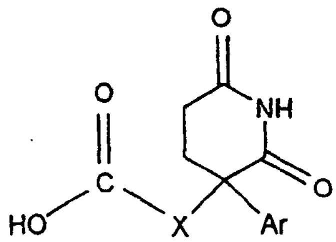

(I)

dans laquelle Ar et X sont tels que définis dans la revendication 1, de ses sels et de ses énantiomères, **caractérisé en ce que** :

on cyclise un composé de formule (III):

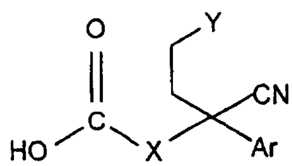

(III)

dans laquelle Ar et X sont tels que définis ci-dessus et Y est un groupe cyano ou carboxy ;
et on isole le composé ainsi obtenu de formule (I) sous forme d'un de ses sels ou sous sa forme acide que l'on transforme éventuellement en un de ses sels.

**6.** Procédé de préparation d'un glutarimide de formule (I) :

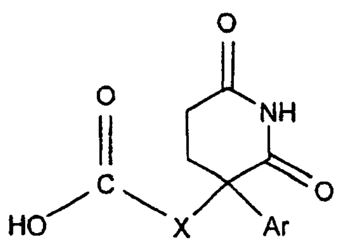

(I)

dans laquelle Ar et X sont tels que définis dans la revendication 1, de ses sels et de ses énantiomères, **caractérisé en ce que** :

(a) on saponifie le ou les groupe(s) ester(s) d'un arylacétonitrile $\alpha,\alpha$-disubstitué de formule (II) :

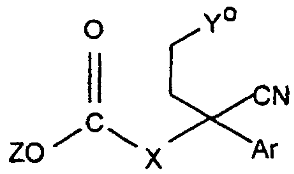 (II)

dans laquelle Ar et X sont tels que définis dans la revendication 1, Y° est un groupe cyano ou COOAlk, Z est l'hydrogène ou Alk, Alk étant un groupe $(C_1-C_3)$alkyle, et au moins un des groupes COOZ et Y° étant COOAlk, (b) on cyclise le composé de formule (III) :

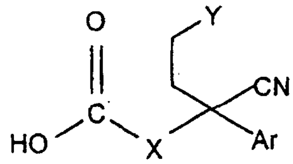 (III)

dans laquelle Ar et X sont tels que définis ci-dessus et Y est un groupe cyano ou carboxy ;

et on isole le composé ainsi obtenu sous forme d'un de ses sels ou sous sa forme acide que l'on transforme éventuellement en un de ses sels.

**7.** Procédé selon la revendication 5, **caractérisé en ce que** comme produit de départ on utilise un composé de formule (III) dans laquelle X, Y et Z sont tels que définis dans la revendication 5 et Ar représente 3,4-difluorophényle ou 3,4-dichlorophényle.

**8.** Procédé selon la revendication 6, **caractérisé en ce que** comme produit de départ on utilise un composé de formule (II) dans laquelle X, Y°, et Z sont tels que définis dans la revendication 6 et Ar représente 3,4-difluorophényle ou 3,4-dichlorophényle.

**9.** Procédé selon la revendication 6 **caractérisé en ce que** comme produit de départ on utilise un composé de formule (II) dans laquelle X est éthylène, Y° est COOAlk et Z est Alk, Alk étant $(C_1-C_3)$alkyle et le composé de départ de l'étape (b) a la formule (III) dans laquelle X est éthylène et Y est carboxy.

**10.** Procédé selon la revendication 9 **caractérisé en ce que** comme composé de départ on utilise le 4-cyano-4-(3,4-dichlorophényl)heptanedioate de méthyle.

**11.** Procédé selon la revendication 1 **caractérisé en ce que** comme produit de départ on utilise un composé de formule (III) dans laquelle X est méthylène, Y est cyano et Ar est tel que défini dans la revendication 1.

**12.** Procédé selon la revendication 2 **caractérisé en ce que** comme produit de départ on utilise l'acide 3-(3,4-dichlorophényl)-3,5-dicyanopentanoïque soit sous forme racémique, soit sous sa forme (-).

**13.** Composé de formule (III'):

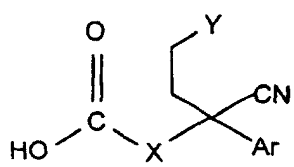 (III')

dans laquelle Ar représente un phényle non substitué ou substitué une ou plusieurs fois par un des substituants choisis parmi un atome d'halogène, un hydroxyle, un $(C_1-C_4)$alcoxy, un trifluorométhyle, un $(C_1-C_4)$alkyle, lesdits

substituants étant identiques ou différents ; un groupe pyridyle ; un groupe thiényle , X est méthylène ou éthylène, et Y est un groupe cyano ou carboxy, et ses sels, sous réserve que :

- lorsque X est éthylène, Ar est différent d'un phényle non susbtitué ; et
- lorsque X est éthylène, Ar est différent d'un phényle non substitué ou d'un phényle substitué en position 2 par un méthyle ou en position 4 par un atome de chlore, un atome de brome, un atome de fluor, un méthyle ou un méthoxy ; ou d'un phényle substitué en position 3 par un méthoxy, ou 3,4-disubstitué par un méthoxy.

**14.** Composé de formule (III") selon la revendication 13 sous forme d'un de ses sels optiquement actifs, d'un de ses énantiomères ou d'un sel de ceux-ci.

**15.** Composé selon la revendication 13 ou 14 dans laquelle Ar est 3,4-dichlorophényle ou 3,4-difluorophényle.

**16.** Composé selon la revendication 13 ou 14, qui est l'acide 3-(3,4-dichlorophényl)-3,5-dicyanopentanoïque et ses sels.

**17.** Composé selon la revendication 16, qui est l'acide 3-(3,4-dichlorophényl)-3,5-dicyanopentanoïque, sous forme d'un de ses sels avec des amines optiquement actives.

**18.** Composé selon la revendication 17, qui est le sel de I-cinchonidine de l'acide 3-(3,4-dichlorophényl)-3,5-dicyano-pentanoïque.

**19.** Composé selon la revendication 14, qui l'acide (-)-3-(3,4-dichlorophényl)-3,5-dicyanopentanoïque.

**20.** Composé selon la revendication 13 ou 14, qui est l'acide 4-cyano-4-(3,4-dichlorophényl)heptanedioïque et ses sels.

**21.** Utilisation d'un glutarimide de formule (I):

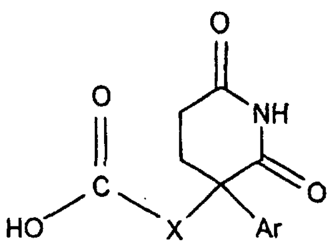

(I)

dans laquelle Ar et X sont tels que définis dans la revendication 1, de ses sels et de ses énantiomères pour la préparation d'une pipéridine 3,3-disubstituée de formule (A) :

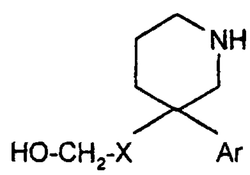

(A)

et de ses sels.

**22.** Utilisation selon la revendication 21 pour la préparation d'un composé de formule (A) dans laquelle Ar est 3,4-difluorophényle ou 3,4-dichlorophényle.

**23.** Utilisation selon la revendication 22 pour la préparation de la (+)-3-(3,4-dichlorophényl)-3-(3-hydroxypropyl)pipéridine.

**24.** Utilisation selon la revendication 22 pour la préparation de la (-)-3-(3,4-dichlorophényl)-3-(2-hydroxyéthyl)pipéridine.

**25.** Procédé pour la préparation d'une pipéridine 3,3-disubstituée de formule (A) :

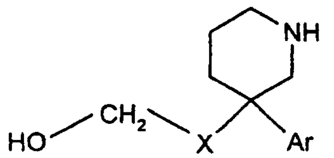

(A)

dans laquelle Ar et X sont tels que définis dans la revendication 1, et de ses sels avec des acides inorganiques ou organiques, **caractérisé en ce qu'**on soumet à une réaction de réduction un glutarimide de formule (I) :

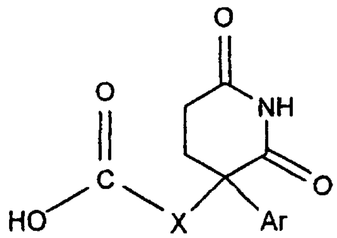

(I)

telle que définie dans la revendication 1, et on isole ladite pipéridine 3,3-disubstituée sous forme de base ou d'un de ses sels ou on transforme la base libre dans un de ses sels.

**26.** Procédé selon la revendication 21, **caractérisé en ce que** la réaction de réduction est effectuée avec du borane.

**27.** Procédé selon la revendication 26 **caractérisé en ce que** comme composé de départ on utilise un glutarimide selon la revendication 2.

**Patentansprüche**

**1.** Glutarimid der Formel (I):

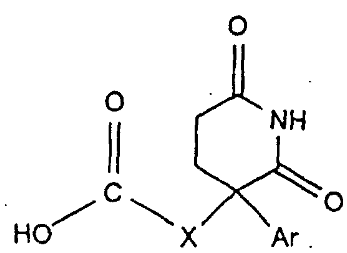

(I)

worin Ar Phenyl, gegebenenfalls ein- oder mehrmals substituiert durch einen Substituenten ausgewählt aus einem Halogenatom, Hydroxyl, ($C_1$-$C_4$)-Alkoxy, Trifluormethyl, ($C_1$-$C_4$)-Alkyl, wobei die Substituenten gleich oder verschieden sind; Pyridyl oder Thienyl darstellt und X Methylen oder Ethylen darstellt, und die Salze desselben mit der Maßgabe, dass

- wenn X Methylen ist, Ar kein unsubstituiertes Phenyl ist; und
- wenn X Ethylen ist, Ar kein unsubstituiertes oder in Position 2 durch Methyl oder in Position 4 durch ein Chloratom, ein Bromatom, ein Fluoratom, Methyl oder Methoxy substituiertes Phenyl ist.

**2.** Glutarimid der Formel (I) nach Anspruch 1 in Form eines seiner optisch aktiven Salze, eines seiner Enantiomere

oder eines Salzes derselben.

**3.** Glutarimid nach den Ansprüchen 1 oder 2, **dadurch gekennzeichnet, dass** in der Formel (I) Ar 3,4-Difluorphenyl oder 3,4-Dichlorphenyl darstellt.

**4.** Glutarimid nach einem der Ansprüche 1 bis 3 in Form eines seiner Salze mit optisch aktiven Aminen.

**5.** Verfahren zur Herstellung eines Glutarimids der Formel (I):

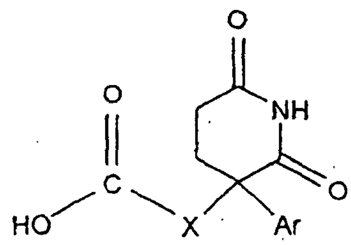

(I)

worin Ar und X wie in Anspruch 1 definiert sind, der Salze und der Enantiomere desselben, **dadurch gekennzeichnet, dass** eine Verbindung der Formel (III)

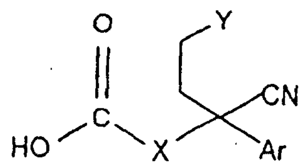

(III)

worin Ar und X wie oben definiert sind und Y Cyano oder Carboxy darstellt, zyklisiert wird und die so erhaltene Verbindung der Formel (I) in Form eines ihrer Salze oder in ihrer sauren Form isoliert wird, wobei letztere gegebenenfalls in ein Salz übergeführt wird.

**6.** Verfahren zur Herstellung eines Glutarimids der Formel (I):

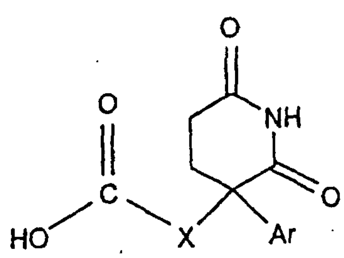

(I)

worin Ar und X wie in Anspruch 1 definiert sind, der Salze und der Enantiomere desselben, **dadurch gekennzeichnet, dass**:

(a) die Estergruppe(n) eines $\alpha,\alpha$-disubstituierten Arylacetonitrils der Formel (II):

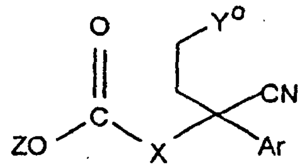

(II)

worin Ar und X wie in Anspruch 1 definiert sind, Y° Cyano oder COOAlk darstellt, Z Wasserstoff oder Alk darstellt, wobei Alk ($C_1$-$C_3$)-Alkyl ist, und mindestens eine der Gruppen COOZ und Y° COOAlk darstellt, verseift wird,

(b) die Verbindung der Formel (III):

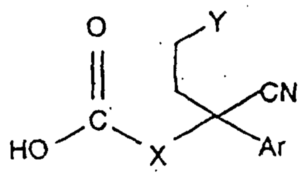

(III)

worin Ar und X wie oben definiert sind und Y Cyano oder Carboxy darstellt, zyklisiert wird und die so erhaltene Verbindung in Form eines ihrer Salze oder in ihrer sauren Form isoliert wird, wobei letztere gegebenenfalls in ein Salz übergeführt wird.

7. Verfahren nach Anspruch 5, **dadurch gekennzeichnet, dass** als Ausgangsprodukt eine Verbindung der Formel (III) verwendet wird, worin X, Y und Z wie in Anspruch 5 definiert sind und Ar 3,4-Difluorphenyl oder 3,4-Dichlorphenyl darstellt.

8. Verfahren nach Anspruch 6, **dadurch gekennzeichnet, dass** als Ausgangsprodukt eine Verbindung der Formel (II) verwendet wird, worin X, Y° und Z wie in Anspruch 6 definiert sind und Ar 3,4-Difluorphenyl oder 3,4-Dichlorphenyl darstellt.

9. Verfahren nach Anspruch 6, **dadurch gekennzeichnet, dass** als Ausgangsprodukt eine Verbindung der Formel (II) verwendet wird, worin X Ethylen darstellt, Y° COOAlk darstellt und Z Alk darstellt, wobei Alk ($C_1$-$C_3$)-Alkyl ist, und die Ausgangsverbindung von Schritt (b) die Formel (III) hat, worin X Ethylen ist und Y Carboxy ist.

10. Verfahren nach Anspruch 9, **dadurch gekennzeichnet, dass** als Ausgangsverbindung Methyl-4-cyano-4-(3,4-dichlorphenyl)-heptandioat verwendet wird.

11. Verfahren nach Anspruch 1, **dadurch gekennzeichnet, dass** als Ausgangsprodukt eine Verbindung der Formel (III) verwendet wird, worin X Methylen darstellt, Y Cyano darstellt und Ar wie in Anspruch 1 definiert ist.

12. Verfahren nach Anspruch 2, **dadurch gekennzeichnet, dass** als Ausgangsprodukt 3-(3,4-Dichlorphenyl)-3,5-dicyanopentansäure entweder in racemischer Form oder in der (-)-Form verwendet wird.

13. Verbindung der Formel (III'):

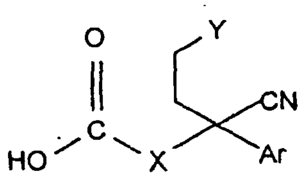

(III')

worin Ar Phenyl, gegebenenfalls ein- oder mehrmals substituiert durch einen Substituenten ausgewählt aus einem

Halogenatom, Hydroxyl, ($C_1$-$C_4$)-Alkoxy, Trifluormethyl, ($C_1$-$C_4$)-Alkyl, wobei die Substituenten gleich oder verschieden sind; Pyridyl oder Thienyl darstellt, X Methylen oder Ethylen darstellt und Y Cyano oder Carboxy darstellt, und die Salze derselben-mit der Maßgabe, dass

- wenn X Methylen ist, Ar kein unsubstituiertes Phenyl ist; und
- wenn X Ethylen ist, Ar kein unsubstituiertes oder in Position 2 durch ein Methyl oder in Position 4 durch ein Chloratom, ein Bromatom, ein Fluoratom, Methyl oder Methoxy substituiertes Phenyl oder in Position 3 durch Methoxy substituiertes oder durch Methoxy 3,4-subsituiertes Phenyl ist.

**14.** Verbindung der Formel (III') nach Anspruch 13 in Form eines ihrer optisch aktiven Salze, eines ihrer Enantiomere oder eines Salzes derselben.

**15.** Verbindung nach Anspruch 13 oder 14, worin Ar 3,4-Dichlorphenyl oder 3,4-Difluorphenyl darstellt.

**16.** Verbindung nach Anspruch 13 oder 14 in Form von 3-(3,4-Dichlorphenyl)-3,5-dicyanopentansäure und Salzen derselben.

**17.** Verbindung nach Anspruch 16 in Form eines der Salze von 3-(3,4-Dichlorphenyl)-3,5-dicyanopentansäure mit optisch aktiven Aminen.

**18.** Verbindung nach Anspruch 17 in Form des 3-(3,4-Dichlorphenyl)-3,5-dicyanopentansäure-1-Cinchonidinsalzes.

**19.** Verbindung nach Anspruch 14 in Form der (-)-3-(3,4-Dichlorphenyl)-3,5-dicyanopentansäure.

**20.** Verbindung nach Anspruch 13 oder 14 in Form von 4-Cyano-4-(3,4-Dichlorphenyl)-heptandisäure und Salzen derselben.

**21.** Verwendung eines Glutarimids der Formel (I):

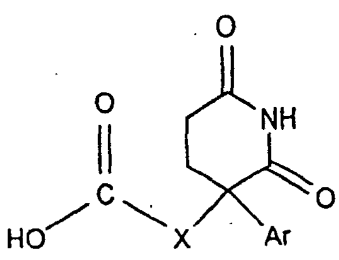

(I)

worin Ar und X wie in Anspruch 1 definiert sind, der Salze desselben und seiner Enantiomere zur Herstellung eines 3,3-disubstituierten Piperidins der Formel (A):

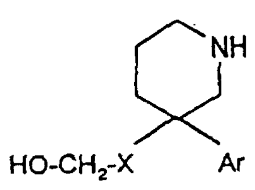

(A)

und der Salze desselben.

**22.** Verwendung nach Anspruch 21 zur Herstellung einer Verbindung der Formel (A), worin Ar 3,4-Difluorphenyl oder 3,4-Dichlorphenyl darstellt.

**23.** Verwendung nach Anspruch 22 zur Herstellung von (+)-3-(3,4-Dichlorphenyl)-3-(3-hydroxypropyl)-piperidin.

**24.** Verwendung nach Anspruch 22 zur Herstellung von (-)-3-(3,4-Dichlorphenyl)-3-(2-hydroxyethyl)-piperidin.

**25.** Verfahren zur Herstellung eines 3,3-disubstituierten Piperidins der Formel (A):

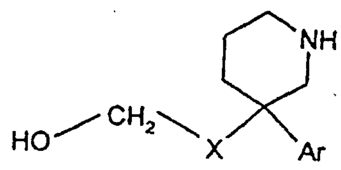

(A)

worin Ar und X wie in Anspruch 1 definiert sind, und der Salze desselben mit anorganischen oder organischen Säuren, **dadurch gekennzeichnet, dass** ein Glutarimid der Formel (I)

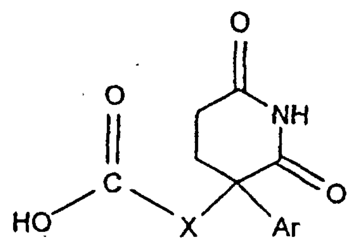

(I)

wie in Anspruch 1 definiert, einer Reduktionsreaktion unterworfen und das 3,3-disubstituierte Piperidin in Form der Base oder eines seiner Salze isoliert oder die freie Base in ein Salz übergeführt wird.

**26.** Verfahren nach Anspruch 21, **dadurch gekennzeichnet, dass** die Reduktionsreaktion mit Bor durchgeführt wird.

**27.** Verfahren nach Anspruch 26, **dadurch gekennzeichnet, dass** als Ausgangsverbindung ein Glutarimid nach Anspruch 2 verwendet wird.

## Claims

**1.** Glutarimide of formula (I):

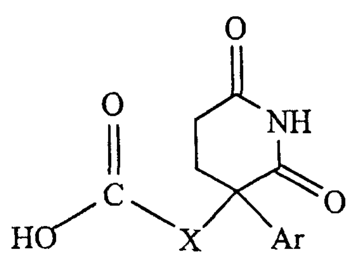

(I)

in which Ar represents a phenyl non substituted or substituted one or many times with one of the substituents selected from a halogen atom, a hydroxyl, a ($C_1$-$C_4$)alkoxy, a trifluoromethyl, a ($C_1$-$C_4$)alkyl, said substituents being identical or different ; a pyridyl group ; a thienyl group and X is methylene or ethylene, and its salts, provided that:

- when X is methylene, Ar is different from a non substituted phenyl; and
- when X is ethylene, Ar is different from a non substituted phenyl or a phenyl substituted in position 2 with a methyl or in position 4 with a chlorine atom, a fluorine atom, a bromine atom, a methyl or a methoxy.

**2.** Glutarimide of formula (I) according to claim 1 in the form of one of its optically active salts, one of its enantiomers, or a salt thereof.

**3.** Glutarimide according to claims 1 or 2, **characterised in that** in the formula (I) Ar is 3,4-difluorophenyl or 3,4-dichlorophenyl.

**4.** Glutarimide according to one of claims 1 to 3 in the form of one of its salts with optically active amines.

**5.** Method for the preparation of a glutarimide of formula (I):

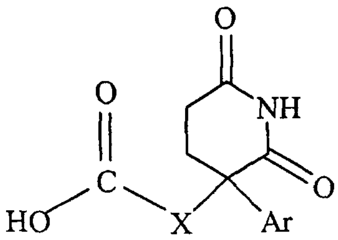

(I)

in which Ar and X are such as defined in claim 1, of its salts and of its enantiomers, **characterised in that** :
a compound of formula (III) :

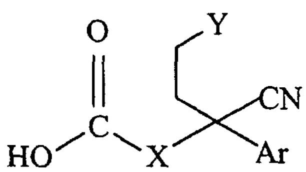

(III)

in which Ar and X are such as defined above and Y is a cyano or carboxy group is cyclised;
and the compound thus obtained of formula (I) is isolated in the form of one of its salts or its acid form which is optionally converted into one of its salts.

**6.** Method for the preparation of a glutarimide of formula (I):

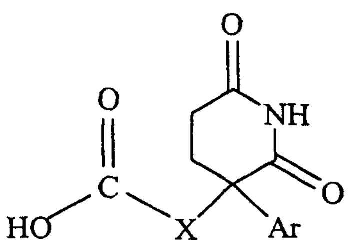

(I)

in which Ar and X are such as defined in claim 1, of its salts and of its enantiomers, **characterised in that**:

(a) the ester group(s) of an α,α-disubstituted arylacetonitrile of formula (II) :

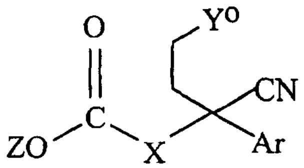

(II)

in which Ar and X are such as defined in claim 1, Y$^{o}$ is a cyano or COOAlk group, Z is hydrogen or Alk, Alk being a ($C_1$-$C_3$)alkyl group, and at least one of the COOZ and Y° groups being COOAlk is (are) saponified, (b) the compound of formula (III):

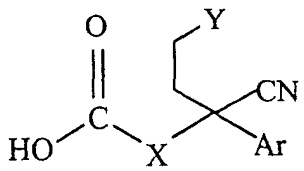

(III)

in which Ar and X are such as defined above and Y is a cyano or carboxy group is cyclised; and the compound thus obtained is isolated in the form of one of its salts or in its acid form which is optionally converted into one of its salts.

**7.** Method according to claim 5, **characterised in that** a compound of formula (III) is used as starting product in which X, Y and Z, are such as defined in claim 5 and Ar represents 3,4-difluorophenyl or 3,4-dichlorophenyl.

**8.** Method according to claim 6, **characterised in that** a compound of formula (II) is used as starting product in which X, Y$^{o}$, and Z are such as defined in claim 6 and Ar represents 3,4-difluorophenyl or 3,4-dichlorophenyl.

**9.** Method according to claim 6, **characterised in that** a compound of formula (II) is used as starting product in which X is ethylene, Y$^{o}$ is COOAlk and Z is Alk, Alk being ($C_1$-$C_3$)alkyl and the starting compound of step (b) has the formula (III) in which X is ethylene and Y is carboxy.

**10.** Method according to claim 9, **characterised in that** methyl 4-cyano-4-(3,4-dichlorophenyl)heptanedioate is used as starting product.

**11.** Method according to claim 1, **characterised in that** a compound of formula (III) is used as starting product in which X is methylene, Y is cyano and Ar is such as defined in claim 1.

**12.** Method according to claim 2, **characterised in that** 3-(3,4-dichlorophenyl)-3,5-dicyanopentanoic acid is used as starting product either in the racemic form, or in the (-) form.

**13.** Compound of formula (III') :

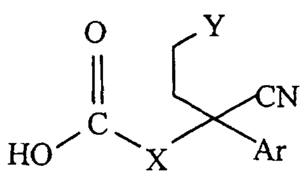

(III')

in which Ar represents a phenyl non substituted or substituted one or many times with one of the substituents selected from a halogen atom, a hydroxyl, a ($C_1$-$C_4$)alkoxy, a trifluoromethyl, a ($C_1$-$C_4$)alkyl, said substituents being identical or different ; a pyridyl group ; a thienyl group, X is methylene or ethylene, and Y is a cyano or carboxy

group, and its salts, provided that :

- when X is ethylene, Ar is different from a non substituted phenyl; and
- when X is ethylene, Ar is different from a non substituted phenyl or a phenyl substituted in position 2 with a methyl or in position 4 with a chlorine atom, a fluorine atom, a bromine atom, a methyl or a methoxy; or from a phenyl substituted in position 3 with a methoxy, or 3,4-disubstituted with a methoxy.

**14.** Compound of formula (III") according to claim 13 in the form of one of its optically active salts, one of its enantiomers or a salt thereof.

**15.** Compound according to claim 13 or 14, in which Ar is 3,4-dichlorophenyl or 3,4-difluorophenyl.

**16.** Compound according to claim 13 or 14, which is 3-(3,4-dichlorophenyl)-3,5-dicyanopentanoic acid and its salts.

**17.** Compound according to claim 16, which is 3-(3,4-dichlorophenyl)-3,5-dicyanopentanoic acid, in the form of one of its salts with optically active amines.

**18.** Compound according to claim 17, which is the 1-cinchonidine salt of 3-(3,4-dichlorophenyl)-3,5-dicyano-pentanoic acid.

**19.** Compound according to claim 14, which is (-)-3-(3,4-dichlorophenyl)-3,5-dicyanopentanoic acid.

**20.** Compound according to claim 13 or 14, which is 4-cyano-4-(3,4-dichlorophenyl)heptanedioic acid and its salts.

**21.** Use of a glutarimide of formula (I):

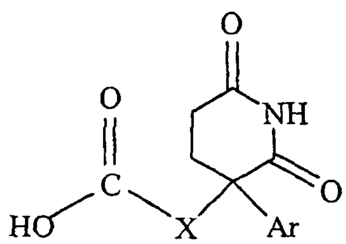

(I)

in which Ar and X are such as defined in claim 1, of its salts and of its enantiomers for the preparation of a 3,3-disubstituted piperidine of formula (A):

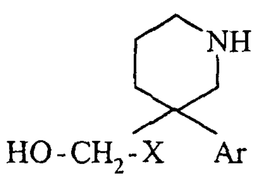

(A)

and of its salts.

**22.** Use according to claim 21 for the preparation of a compound of formula (A) in which Ar is 3,4-difluorophenyl or 3,4-dichlorophenyl.

**23.** Use according to claim 22 for the preparation of (+)-3-(3,4-dichlorophenyl)-3 -(3-hydroxypropyl)piperidine.

**24.** Use according to claim 22 for the preparation of (-)-3-(3,4-dichlorophenyl)-3-(2-hydroxyethyl)piperidine.

**25.** Method for the preparation of a 3,3-disubstituted piperidine of formula (A):

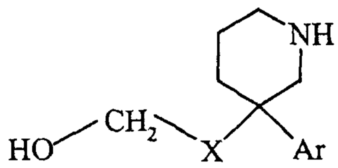

(A)

in which Ar and X are such as defined in claim 1, and of its salts with inorganic or organic acids, **characterised in that** a glutarimide of formula (I) is submitted to a reduction reaction :

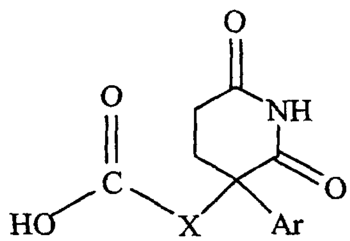

(I)

such as defined in claim 1, and said 3,3-disubstituted piperidine is isolated in the form of a base or one of its salts or the free base is converted into one of its salts.

**26.** Method according to claim 21, **characterised in that** the reduction reaction is carried out with borane.

**27.** Method according to claim 26, **characterised in that** a glutarimide according to claim 2 is used as starting compound.